(19) Europäisches Patentamt
European Patent Office
Office européen des brevets

(11) EP 2 366 355 A1

(12) EUROPÄISCHE PATENTANMELDUNG

(43) Veröffentlichungstag:
21.09.2011 Patentblatt 2011/38

(51) Int Cl.:
A61B 18/20 (2006.01)    A61C 1/00 (2006.01)

(21) Anmeldenummer: 10002770.5

(22) Anmeldetag: 16.03.2010

(84) Benannte Vertragsstaaten:
AT BE BG CH CY CZ DE DK EE ES FI FR GB GR
HR HU IE IS IT LI LT LU LV MC MK MT NL NO PL
PT RO SE SI SK SM TR
Benannte Erstreckungsstaaten:
AL BA ME RS

(71) Anmelder: Quantel Derma GmbH
91058 Erlangen (DE)

(72) Erfinder: Fischer, Dietmar, Dr.
91056 Erlangen (DE)

(74) Vertreter: von Hellfeld, Axel
Wuesthoff & Wuesthoff
Patent- und Rechtsanwälte
Schweigerstrasse 2
81541 München (DE)

(54) **Medizinische Behandlungsvorrichtung mit Laserimpulsen im Femtosekundenbereich**

(57) Die Erfindung offenbart eine Hautbehandlungsvorrichtung, eine Dentalbehandlungsvorrichtung und eine chirurgische Vorrichtung zur Behandlung eines inneren Organs mit einer Laserimpulsquelle zum Erzeugen von Laserimpulsen (38) mit einer Impulslänge, die sich bevorzugt im Femtosekundenbereich befindet, und einem optischen Pfad zum Richten der Laserimpulse von der Laserimpulsquelle auf die Oberfläche der Haut (30), des Zahnfleisches, des Zahns oder des inneren Organs. Die Laserimpulse werden so auf die Oberfläche der Haut, des Zahnfleisches, des Zahns oder des inneren Organs gerichtet, dass die Impulse in der Haut, im Zahnfleisch, im Zahn oder im inneren Organ fokussiert werden, so dass sie unter der Oberfläche der Haut, des Zahnfleisches, des Zahns oder des inneren Organs einen laserinduzierten optischen Durchbruch (40) erzeugen, wobei die Laserimpulse an der Oberfläche der Haut (30), des Zahnfleisches, des Zahns oder des inneren Organs eine größere Querschnittsfläche als am Ort des laserinduzierten optischen Durchbruchs (40) aufweisen.

Fig. 3

EP 2 366 355 A1

**Beschreibung**

Die vorliegende Erfindung betrifft eine Hautbehandlungsvorrichtung, eine Dentalbehandlungsvorrichtung sowie eine chirurgische Vorrichtung zur Behandlung eines inneren Organs mit Laserimpulsen, die zwischen der Oberfläche der Haut, des Zahnfleisches, eines Zahns sowie des inneren Organs und dem Ort des laserinduzierten optischen Durchbruchs unter der Oberfläche fokussiert werden.

In der Dermatologie werden Lasersysteme, beispielsweise zur Entfernung von Tätowierungen, zum Veröden von Krampfadern, zum Entfernen unerwünschter Pigmentierungen sowie zur Epilation, weit verbreitet verwendet. Die derzeit im Bereich der Dermatologie verwendeten Lasersysteme haben den Nachteil, dass sie beim Erzeugen einer Heilwirkung in der Haut auch die Hautoberfläche schädigen. Beispielsweise können bei einer Behandlung in tieferen Hautschichten die äußeren Hautschichten der Epidermis, beispielsweise das Stratum Disjunctum, das Stratum Conjunktum sowie das Stratum Lucidum, geschädigt werden, was unerwünscht ist, weil der Heilungsprozess verzögert wird, eine offene Wunde mit entsprechendem Infektionsrisiko entsteht und die Schädigung der äußeren Schichten der Epidermis vom Patienten als unerwünschte Nebenwirkung betrachtet wird.

Die Erfindung stellt sich zur Aufgabe, eine medizinische Behandlungsvorrichtung zu schaffen, mit der die Haut, das Zahnfleisch, der Zahn oder ein inneres Organ in einer vorbestimmten Tiefe mit Laserstrahlung behandelt werden kann, ohne die äußeren Schichten der Haut, des Zahnfleisches, des Zahns oder des inneren Organs zu schädigen.

Die Aufgabe der Erfindung wird durch eine Hautbehandlungsvorrichtung mit einer Laserimpulsquelle zum Erzeugen von Laserimpulsen und einem optischen Pfad zum Richten der Laserimpulse von der Laserimpulsquelle auf die Haut gelöst, wobei die Laserimpulse so auf die Haut appliziert werden, dass die Impulse in der Haut fokussiert werden, so dass sie unter der Hautoberfläche einen laserinduzierten Durchbruch erzeugen, wobei die Laserimpulse an der Oberfläche der Haut eine größere Querschnittsfläche als am Ort des laserinduzierten optischen Durchbruchs aufweisen. Die Laserimpulsquelle erzeugt vorzugsweise Laserimpulse mit einer Impulsdauer im Femtosekundenbereich. Der Ausdruck Femtosekundenbereich ist so zu verstehen, dass sich die Impulsdauer im Bereich von wenigen Femtosekunden bis zu 1000 Pikosekunden bewegt.

Die Aufgabe der Erfindung wird auch durch eine Dentalbehandlungsvorrichtung mit einer Laserimpulsquelle zum Erzeugen von Laserimpulsen und einem optischen Pfad zum Richten der Laserimpulse von der Laserimpulsquelle auf das Zahnfleisch oder einen Zahn gelöst, wobei die Laserimpulse so auf das Zahnfleisch oder den Zahn appliziert werden, dass die Impulse im Zahnfleisch oder im Zahn fokussiert werden, so dass sie unter der Zahnfleischoberfläche oder der Zahnoberfläche einen laserinduzierten optischen Durchbruch erzeugen, wobei die Laserimpulse an der Oberfläche des Zahnfleisches oder des Zahns eine größere Querschnittsfläche als am Ort des laserinduzierten optischen Durchbruchs aufweisen. Die Laserimpulsquelle erzeugt vorzugsweise Laserimpulse mit einer Impulsdauer im Femtosekundenbereich. Der Ausdruck Femtosekundenbereich ist so zu verstehen, dass sich die Impulsdauer im Bereich von wenigen Femtosekunden bis zu 1000 Pikosekunden bewegt.

Die Aufgabe der Erfindung wird auch durch eine chirurgische Vorrichtung zur Behandlung eines inneren Organs mit einer Laserimpulsquelle zum Erzeugen von Laserimpulsen und einem optischen Pfad zum Richten der Laserimpulse von der Laserimpulsquelle auf die Oberfläche des inneren Organs gelöst, wobei die Laserimpulse so auf die Oberfläche eines inneren Organs appliziert werden, dass die Impulse im inneren Organ fokussiert werden, so dass sie unter der Oberfläche des inneren Organs einen laserinduzierten optischen Durchbruch erzeugen, wobei die Laserimpulse an der Oberfläche des inneren Organs eine größere Querschnittsfläche als am Ort des laserinduzierten optischen Durchbruchs aufweisen. Die Laserimpulsquelle erzeugt vorzugsweise Laserimpulse mit einer Impulsdauer im Femtosekundenbereich. Der Ausdruck Femtosekundenbereich ist so zu verstehen, dass sich die Impulsdauer im Bereich von wenigen Femtosekunden bis zu 1000 Pikosekunden bewegt.

Der Begriff Organ, wie er im Folgenden verwendet wird, umfasst die Haut, eine Schleimhaut, die Mundschleimhaut, das Zahnfleisch, einen Zahn sowie ein inneres Organ. Das innere Organ kann beispielsweise ein Darm, die Prostata, der Magen, die Speiseröhre, die Gebärmutter, der Gebärmutterhals, die Lunge, der Rachen, der Kehlkopf, das Gehirn, das Herz und dergleichen sein. Die zuvor beschriebenen Vorrichtungen haben den Vorteil, dass die Oberfläche des Organs nicht geschädigt wird, da die Laserimpulse an der Oberfläche eine vergleichsweise große Querschnittsfläche und somit eine niedrige Energiedichte aufweisen und erst auf der Strecke zum Ort des laserinduzierten optischen Durchbruchs fokussiert werden, wo die Laserimpulse eine entsprechend höhere Energiedichte aufweisen. Dadurch kann eine Schädigung der äußeren Gewebeschichten des Organs vermieden werden, wodurch der Heilungsprozess beschleunigt wird und weniger unerwünschte Nebenwirkungen für den Patienten auftreten.

Die Photodisruption durch den laserinduzierten optischen Durchbruch kann einen Durchmesser von etwa 0,1 $\mu m$ bis etwa 50 $\mu m$ haben. Da die Leistungsdichte der Laserimpulse an der Oberfläche des Organs niedriger ist, wird die Oberfläche des Organs nicht geschädigt. Aufgrund der kurzen Laserimpulse kann eine Übertragung der Wärme an umgebendes Gewebe weitestgehend vermieden werden. Die Eindringtiefe kann durch die Wahl der Wellenlänge der Laserimpulse und durch die Wahl der Spotgröße bzw. Fleckgröße, d. h. der Querschnittsfläche der auf die Oberfläche des Organs auftreffenden Laserimpulse, sowie des Fokussierungswinkels eingestellt werden. Die Größe des durch den laserinduzierten Durchbruch veränderten Gewebebereichs kann mittels der Spitzenleistung und der Impulsdauer bestimmt werden. Die eingangs beschriebene Hautbehandlungsvorrichtung kann verwendet werden, um Fettzellen in der Subkutis gezielt zu zerstören, um Cellulitis zu behandeln oder die Dicke von Fettschichten nicht-invasiv im Rahmen des so genannten "Body Shaping" zu reduzieren. Die Hautbehandlungsvorrichtung kann auch zur Hautverjüngung (Skin Rejunivation) verwendet werden, bei der Falten geglättet werden und die Haut gestrafft wird. Die Hautbehandlungsvorrichtung erzeugt ähnlich wie bei der so genannten fraktionalen Hautbehandlung kleine Wunden in der Haut, die der Körper dann repariert und hierbei neues Kollagen erzeugt. Im Gegensatz zur herkömmlichen fraktionalen Behandlung verursacht die Hautbehandlungsvorrichtung jedoch keine Schädigung und/oder offene Wunde in der Epidermis.

Die Laserimpulse können in einer Tiefe von etwa 30 $\mu$m bis etwa 10 mm unterhalb der Oberfläche des Organs fokussiert werden, um dort die laserinduzierten Durchbrüche bzw. Photodisruptionen auszulösen. Beispielsweise ist die Epidermis am Augenlied lediglich etwa 30 $\mu$m und an der Fußsohle etwa 4 mm dick.

Ein weiterer Anwendungsbereich ist die gezielte Zerstörung von unter der obersten Hautschicht liegenden unerwünschten Läsionen. Beispielsweise können Tumorgewebe, pigmentierte Läsionen, beispielsweise Pigmentflekken, Melasma, Tätowierungen und dergleichen sowie unpigmentierte Läsionen (Xanthelasmen, Nävi etc.) behandelt werden.

Die chirurgische Vorrichtung kann im Bereich der inneren Medizin, beispielsweise der Urologie, Gynäkologie, Gastroenterologie etc. angewendet werden. Mit der chirurgischen Vorrichtung kann Gewebe unter der obersten Gewebeschicht entfernt werden, beispielsweise ein Tumor oder ein Karzinom.

Durch die Hautbehandlungsvorrichtung können ferner Fettzellen im Subkutis zerstört oder beschädigt werden, wodurch deren Anzahl und der Fettgehalt der Haut reduziert wird, was bei der Fettreduzierung und Behandlung von Cellulitis erwünscht ist. Die oberste Schicht des Subkutis enthält so genannte stehende Fettzellenkammern bzw. läppchenartig angeordnetes Fettgewebe, die voneinander durch Bindegewebe getrennt sind. Das Beschädigen oder Zerstören dieses Bindegewebes erlaubt den Fettzellen, sich gleichmäßiger unter der Epidermisschicht zu verteilen. Auch bei dieser Vorgehensweise ist die Hautbehandlungsvorrichtung zur Behandlung von Cellulitis geeignet. Mit der Hautbehandlungsvorrichtung können auch Haarwurzeln und Blutgefäße behandelt werden. Durch geeignete Wahl der Behandlungstiefe und der Wellenlänge kann die Hautbehandlungsvorrichtung auch zur Haarentfernung, Aderverödung oder Gefäßentfernung verwendet werden. Ebenso könnte Akne behandelt werden, indem die Laserimpulse auf die Talgdrüsen im Korium gerichtet werden. Beschädigte Talgdrüsen erzeugen weniger Talg (Sebum). Eine hohe oder zu hohe Sebumproduktion ist eine der Ursachen für den Ausbruch von Akne Vulgaris oder anderer Akne-Arten. Die Laserimpulse können auch auf das Hautkollagen gerichtet werden, um eine Hautverjüngungsbehandlung durchzuführen, die Haut zu straffen und eine Faltenreduzierungsbehandlung durchzuführen. Das Schädigen oder Zerstören des Kollagens führt zu einer Wundreaktion in der Haut, die dazu führt, dass das Kollagen wieder aufgebaut wird und folglich eine straffere Haut mit weniger Falten und einer glatteren Hautoberfläche erhalten wird.

Es sind verschiedene Arten von Hautkrebs und Vorstufen von Hautkrebs, sog. Präkanzerosen, bekannt. In Abhängigkeit von der Art des Krebses oder der Vorstufe befinden sich die Zellen in unterschiedlichen Schichten der Haut. Mit der Hautbehandlungsvorrichtung können auch krebsbefallene Zellen zerstört werden, indem der Behandlungsstrahl, d. h. die Laserimpulse, auf die entsprechenden Behandlungsbereiche in der jeweiligen Tiefe gerichtet wird bzw. werden.

Die zuvor beschriebene medizinische Vorrichtung, d. h. die Hautbehandlungsvorrichtung, die Dentalbehandlungsvorrichtung und die chirurgische Vorrichtung, können eine Fokussiereinrichtung im optischen Pfad aufweisen, die so eingerichtet ist, dass die Laserimpulse auf der Strecke von der Oberfläche der Haut, des Zahnfleisches, des Zahns oder des inneren Organs zum Ort des laserinduzierten Durchbruchs fokussiert werden. Dadurch kann sichergestellt werden, dass die Laserimpulse in den oberen Gewebeschichten des Organs eine relativ große Querschnittsfläche und somit eine niedrige Energiedichte aufweisen, so dass die oberen Gewebeschichten des Organs nicht beschädigt werden.

Die Fokussiereinrichtung kann eine konvexe Sammellinse sein, die in der Nähe der Oberfläche des Organs angeordnet ist. Die Fleckgröße bzw. Querschnittsfläche der Impulse auf der Oberfläche des Organs kann je nach Pulsdauer und Fokussierungstiefe variieren. Beim der Fokussierung durch die Sammellinse beträgt der Durchmesser bzw. Querschnitt des Flecks bzw. der Querschnittsfläche auf der Oberfläche des Organs zwischen etwa 50 $\mu$m und etwa 5 cm. Der Fleck bzw. die Querschnittsfläche des Impulses müssen nicht rund sein.

Die medizinische Vorrichtung kann so eingerichtet sein, dass die Laserimpulse beim Eintritt in die Haut, in das Zahnfleisch, in den Zahn oder in das Organ eine Intensität aufweisen, die höher als ein Selbstfokussierungsschwellenwert des Hautgewebes, des Zahnfleischgewebes, des Zahns oder des Gewebes des inneren Organs ist, so dass in der Haut, im Zahnfleisch, im Zahn oder im inneren Organ eine Selbstfokussierung auftritt, um den laserinduzierten optischen Durchbruch unter der Hautoberfläche, im Zahnfleisch, im Zahn oder im inneren Organ zu ermöglichen.

Die Selbstfokussierung kann durch den Kerr-Effekt erfolgen. Der Kerr-Effekt ist ein nicht linearer optischer Effekt. Übersteigt die Intensität einen Schwellenwert, ändert sich die Brechzahl in Abhängigkeit von der Intensität. Der Kerr-Effekt ist beispielsweise in Bergmann Schäfer, Lehrbuch der Experimentalphysik, Band 3, Optik, 10. Auflage, p. 940 ff. beschrieben, von wo die folgenden Formeln und Konstantenwerte entnommen wurden. Der Kerr-Effekt lässt sich mit folgender Formel darstellen:

$$n_L = n + \delta J;$$

wobei $n$ die Brechzahl des Mediums unterhalb der kritischen Leistung für die Nichtlinearität, $\delta$ eine stoffabhängige Konstante, $J$ die Intensität in $W/m^2$ und $n_L$ die intensitätsabhängige Brechzahl ist. Für Wasser beträgt $\delta$ etwa $5,4 \times 10^{-20}$ $m^2 W^{-1}$.

Die kritische Leistung $P_k$, ab der eine Selbstfokussierung auftritt, lässt sich mit folgender Formel schätzen:

$$P_k = (\varepsilon_0 \, c_0 \, \lambda_0^2)/(8 \, \pi \, \gamma_L);$$

wobei $P_k$ die kritische Leistung, $\varepsilon_0$ die elektrische Feldkonstante, $c_0$ die Vakuumlichtgeschwindigkeit, $\lambda_0$ die Vakuumwellenlänge und $Y_L$ eine Materialkonstante ist, die für Wasser etwa $0,5 \times 10^{-22}$ $m^2 V^{-2}$ beträgt.

Die kritische Leistung $P_k$, oberhalb der im Wasser die Selbstfokussierung auftritt, beträgt also $1500 \times 10^3$ W.

Zusätzlich treten insbesondere bei kurzen Impulsdauern weitere nicht lineare Effekte auf. Darüber hinaus sind die Stoffkonstanten und die kritische Leistung nur mit hohen Aufwänden zu bestimmen, da die Angaben für $\delta$ und $_{YL}$ stark schwanken, da es schwierig ist, definierte Versuchsbedingungen herzustellen, insbesondere bei kurzen Impulsdauern (siehe Bergmann Schäfer, Lehrbuch der Experimentalphysik, Band 3, Optik, 10. Auflage).

Da der Brechungsindex oberhalb der kritischen Leistung $P_k$ für die Selbstfokussierung von der Intensität abhängt, kann durch Wahl der Intensität der Laserimpulse und des Radius des Lichtflecks auf der Oberfläche der Haut, des Zahnfleisches, des Zahns oder des inneren Organs die Eindringtiefe bestimmt werden. Je höher die Intensität der Laserimpulse ist, desto stärker verändert sich der Brechungsindex, desto stärker werden die Laserimpulse gebrochen und desto näher befindet sich der Ort des laserinduzierten Durchbruchs an der Oberfläche des Organs. Dabei kann folgende Schätzung verwendet werden:

$$d_{LIOB} = (\pi n \, w_0^2)/(\lambda_0 \, (P/P_K - 1)^{1/2}), \, P \geq P_K;$$

wobei $d_{LIOB}$ der Abstand des laserinduzierten Durchbruchs von der Oberfläche des Organs, $J$ die Intensität, $n$ die Brechzahl des Mediums unterhalb der kritischen Leistung für die Nichtlinearität, $w_0$ der Radius des Laserimpulses, $\lambda_0$ die Vakuumwellenlänge, $P_k$ die kritische Leistung, oberhalb der Selbstfokussierung auftritt, und $P$ die Leistung des Laserimpulses ist.

Die Eindringtiefe in das Gewebe hängt ferner von der gewählten Wellenlänge ab. Die Größe des Bereichs, in dem der laserinduzierte Durchbruch im Gewebe auftritt, kann durch die Impulsdauer eingestellt werden.

Die von der Laserimpulsquelle abgegebenen Impulse weisen eine Dauer von etwa 1 fs bis etwa 100 ps, vorzugsweise von etwa 10 fs bis etwa 100 ps, vorzugsweise von etwa 50 fs bis etwa 10 ps, höchstvorzugsweise von etwa 50 fs bis etwa 900 fs auf. Eine derartige Laserquelle ist beispielsweise in der US 7,131,968 B2 beschrieben.

Die Fleckgröße bzw. Querschnittsfläche der Impulse auf der Oberfläche des Organs kann je nach Pulsdauer und Fokussierungstiefe variieren. Bei der Fokussierung durch den Kerr-Effekt beträgt der Durchmesser bzw. Querschnitt des Flecks bzw. der Querschnittsfläche auf der Oberfläche des Organs zwischen etwa 50 $\mu$m und etwa 1 mm. Der Fleck bzw. die Querschnittsfläche des Impulses müssen, wie zuvor erwähnt, nicht rund sein.

Die von der Laserimpulsquelle abgegebenen Impulse weisen eine Wellenlänge von etwa 400 nm bis etwa 10000 nm, vorzugsweise von etwa 700 nm bis etwa 2000 nm, höchstvorzugsweise von etwa 800 nm bis etwa 1500 nm, höchstvorzugsweise von etwa 950 nm bis etwa 1400 nm auf. Durch die Wahl der Wellenlänge können die Eindringtiefe in das Gewebe und/oder die zu behandelnden Gewebeteile bestimmt werden. Als Laserimpulsquelle kann beispielsweise ein Faserlaser, ein Festkörperlaser, ein Yttirbium-basierender Festkörperlaser, ein YAG-basierter Festkörperlaser, ein Cr:Fosterite-Laser, ein Cr:Cunyite-Laser, ein Neodymium-dotierter Lithium-Yttrium-Fluoride-Laser (YLF-Laser) oder ein Neodymium-dotierter Vanadate-Laser ($YVO_4$-Laser), ein Halbleiterlaser, ein Slab-Laser oder ein Dioden-gepumpter-Festkörperlaser verwendet werden. Es kann eine Steuerungseinrichtung vorhanden sein, um einen oder mehrere der folgenden Parameter der Laserimpulsquelle einzustellen und/oder zu messen: Die Impulswiederholrate, die Impulsdauer, die Energie pro Impuls, die Leistung während des Impulses, die durchschnittliche Leistung, die Größe des Lichtfleckes auf der Hautoberfläche bzw. der Organoberfläche, die Tiefe der zu behandelnden Region in der Haut oder im Organ, das Scanmuster, die Fokussierungstiefe und die Wellenlänge.

Der optische Pfad kann eine optische Faser, insbesondere eine hohle optische Faser oder eine photonische Kristallfaser aufweisen. Dadurch können die Laserimpulse von der Laserimpulsquelle zu einem beweglichen Applikator geleitet werden. Der Applikator kann auch an einem Endoskop angeordnet sein, um ein inneres Organ minimalinvasiv zu behandeln.

Der optische Pfad kann zumindest einen beweglichen oder unbeweglichen Spiegel und/oder zumindest eine Linse sowie ein Austrittsfenster aufweisen. Der optische Pfad kann in einem schwenkbaren Arm mit Spiegeln, einem sogenannten Spiegelgelenkarm, angeordnet sein. Der schwenkbare Arm kann die zuvor erwähnte Faser aufweisen. Der optische Pfad kann auch eine beliebige andere Art von Wellenleiter für optische Strahlung aufweisen.

Der optische Pfad kann eine Scaneinrichtung aufweisen, um die Laserimpulse auf einer definierten Fläche zu applizieren. Die Scaneinrichtung eignet sich insbesondere bei einer Hautbehandlung.

Die medizinische Vorrichtung kann eine Messeinrichtung umfassen, die dazu ausgebildet ist, die Eigenschaften der Haut, insbesondere den Hauttyp, die Hauttemperatur und/oder die durch die Laserimpulse verursachten Wirkungen in der Haut, im Zahnfleisch, im Zahn oder im inneren Organ zu messen oder zu ermitteln. Hierdurch kann die geeignete Behandlung des Organs ausgewählt werden und/oder die Behandlung des Organs überwacht werden. Die Laserimpulsquelle kann in Abhängigkeit von den durch die Messeinrichtung ermittelten Messwerten gesteuert werden. Beispielsweise können in Abhängigkeit eines Messwertes die zuvor erwähnten Parameter der Laserimpulsquelle verändert werden. Dies kann automatisch während der Behandlung durchgeführt werden.

Die medizinische Vorrichtung kann eine Abbildungseinrichtung aufweisen, die die Haut mittels optischer Kohärenztomografie oder Ultraschall abbildet. Mittels der Abbildungseinrichtung ist es möglich, den Behandlungsfortschritt manuell oder automatisch zu überwachen.

Die medizinische Behandlungsvorrichtung weist ferner eine Kühleinrichtung zum Kühlen der Haut des Organs vor und/oder während und/oder nach der Behandlung auf. Hierdurch wird der Komfort des Patienten erhöht.

Die medizinische Vorrichtung kann ferner eine Unterdruckerzeugungseinrichtung zum Erzeugen eines Unterdrucks auf der Oberfläche der Haut, des Zahnfleisches, des Zahns oder des inneren Organs aufweisen, um deren Position festzulegen und/oder sie zu strecken. Hierdurch können definierte Operationsbedingungen und größere Eindringtiefen erreicht werden. Zudem reduzieren sich die Schmerzen für den Patienten.

Die medizinische Vorrichtung kann eine Positioniereinrichtung aufweisen, die dazu ausgebildet ist, die Laserimpulse so in der Haut, im Zahnfleisch, im Zahn oder im inneren Organ zu positionieren, dass die Orte der laserinduzierten optischen Durchbrüche einander benachbart sind oder sich überlappen. Diese Vorgehensweise stellt sicher, dass ein Gewebe, beispielsweise ein Tumor oder Karzinom restlos entfernt wird. Die Positionierungseinrichtung kann dazu ausgebildet sein, die Laserimpulse so in der Haut, im Zahnfleisch, im Zahn oder im inneren Organ zu positionieren, dass zwischen den Orten der laserinduzierten optischen Durchbrüche nicht mit Laserimpulsen behandeltes Gewebe vorhanden ist, von dem die Heilung ausgehen kann. Diese Vorgehensweise bietet sich bei einer kosmetischen Behandlung an, um die postoperative Heilung zu beschleunigen, da zwischen den Stellen, die mit laserinduzierten Durchbrüchen behandelt wurden, noch unbehandeltes Gewebe vorhanden ist. Die Positionierungseinrichtung kann durch die zuvor erwähnte Scaneinrichtung implementiert werden.

Die Erfindung betrifft auch die Behandlung der Haut, des Zahnfleisches, eines Zahns oder eines inneren Organs mit Laserimpulsen. Die Laserimpulse werden in der Haut, im Zahnfleisch, im Zahn oder im inneren Organ fokussiert. Die Intensität der Laser-impulse kann so hoch sein, dass in der Haut, im Zahnfleisch, im Zahn oder im inneren Organ eine Selbstfokussierung aufgrund des zuvor beschriebenen Kerr-Effektes auftritt. Das Behandlungsverfahren kann so weitergebildet werden, wie zuvor im Zusammenhang mit der medizinischen Vorrichtung beschrieben wurde. Die Laserimpulse weisen bevorzugt eine Impulsdauer im Femtosekundenbereich auf. Der Ausdruck Femtosekundenbereich ist so zu verstehen, dass sich die Impulsdauer im Bereich von wenigen Femtosekunden bis zu 1000 Pikosekunden bewegt.

Die Erfindung wird jetzt anhand einer beispielhaften Hautbehandlungsvorrichtung mittels der beigefügten Zeichnungen erläutert. Es zeigen:

Fig. 1 eine Hautbehandlungsvorrichtung;

Fig. 2 eine Hautbehandlungsvorrichtung mit einer Messeinrichtung und/oder einer Abbildungseinrichtung;

Fig. 3 einen Schnitt durch die Haut in einem Fall, bei dem die Fokussierung durch die Selbstfokussierung erfolgt; und

Fig. 4 einen Fall, bei dem die Fokussierung durch eine Sammellinse erfolgt.

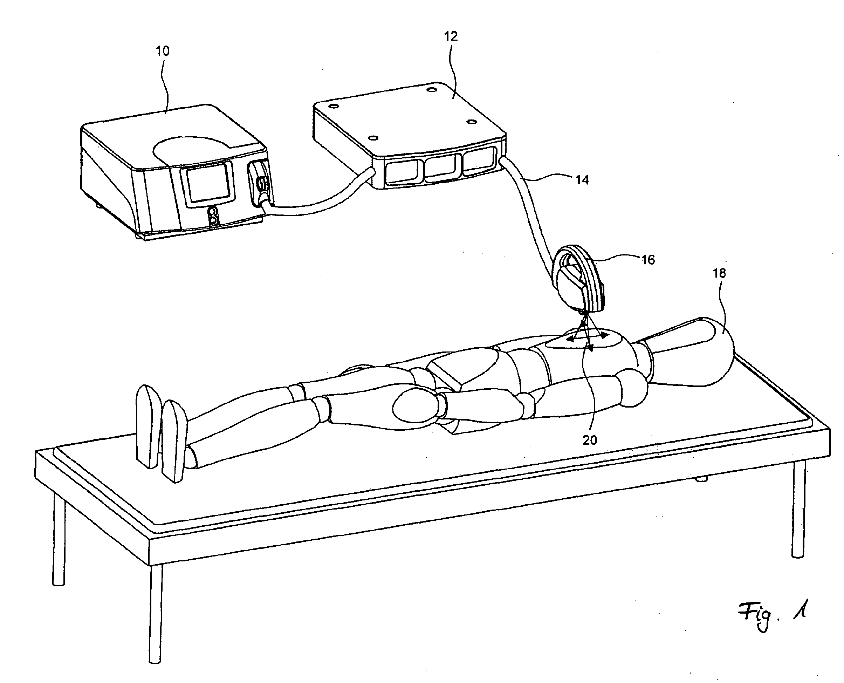

Fig. 1 zeigt eine erste Ausführungsform der Hautbehandlungsvorrichtung. Eine Laserimpulsquelle 12 erzeugt Laserimpulse mit einer Impulsdauer, die sich bevorzugt im Femtosekundenbereich befindet und die über einen optischen Pfad 14 zu einem Applikator 16 geleitet werden. Der optische Pfad 14 kann eine optische Faser aufweisen, so dass der Applikator 16 flexibel zu einem gewünschten Behandlungsort 20 am Patienten 18 bewegt werden kann. Der Applikator 16 weist eine Scaneinrichtung (nicht gezeigt) auf, die die Laserimpulse auf einer definierten Behandlungsfläche 20 appliziert. Die Scaneinrichtung kann zwei bewegliche Spiegel aufweisen.

Der optische Pfad 14 kann zumindest eine Linse aufweisen, um beispielsweise die Größe der Laserimpulse einzustellen. Der optische Pfad kann ein System aus zumindest einem beweglichen oder unbeweglichen Spiegel, zumindest einer Linse und einer optischen Faser aufweisen. In der gezeigten Ausführungsform ist der optische Pfad 14 in einem schwenkbaren flexiblen Arm angeordnet, der zum Behandlungsort schwenkbar ist.

Es ist auch möglich, den Applikator als Handgerät auszubilden, den das medizinische Personal über den Behandlungsbereich bewegt.

Die Hautbehandlungsvorrichtung weist ferner eine Steuerungseinheit 10 auf, in die über eine Benutzerschnittstelle Behandlungsparameter eingegeben werden können.

Es können beispielsweise folgende Behandlungsparameter eingegeben werden: die Impulswiederholrate, die Impulsdauer, die Energie pro Impuls, die Leistung während des Impulses, die durchschnittliche Leistung, die Größe des Lichtfleckes auf der Hautoberfläche, die Tiefe der zu behandelnden Region in der Haut, das Scanmuster, die Fokussierungstiefe, die Wellenlänge, die Behandlungsdauer und die Abmessungen der Behandlungsfläche, die mit der Scaneinrichtung überstrichen werden.

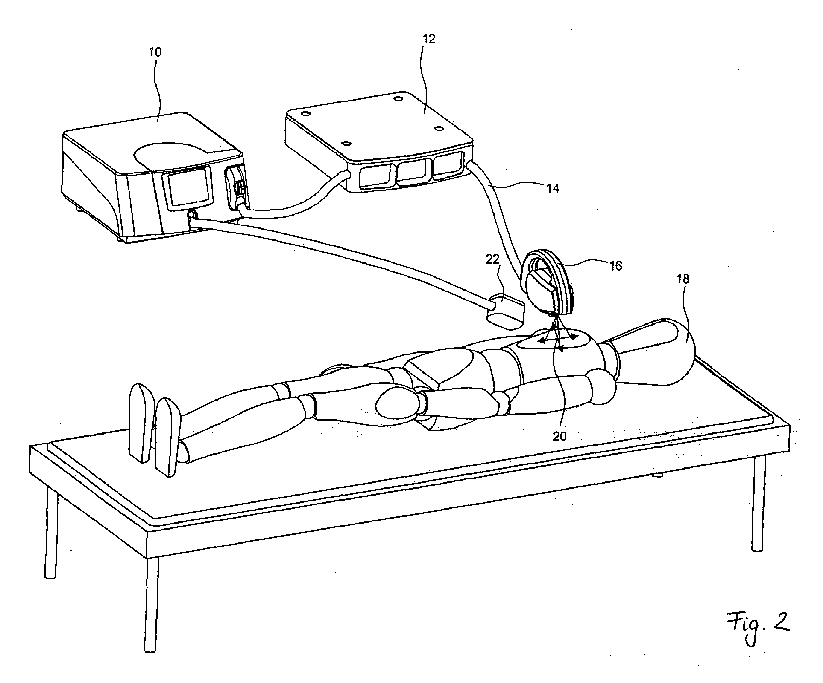

Fig. 2 zeigt eine zweite Ausführungsform der Hautbehandlungsvorrichtung. Die zweite Ausführungsform weist alle Elemente der ersten Ausführungsform auf, die daher nicht erneut beschrieben werden und die mit den gleichen Bezugszeichen wie in Fig. 1 bezeichnet werden. Zusätzlich weist die zweite Ausführungsform eine Sensoreinrichtung 22 auf. Die Sensoreinrichtung 22 kann als Messeinrichtung und/oder als Abbildungseinrichtung ausgebildet sein. Die Sensoreinrichtung 22 kann eine Messeinrichtung aufweisen, die dazu ausgebildet ist, Eigenschaften der Haut, insbesondere den Hauttyp, die Hauttemperatur und/oder die in der Haut durch die Laserimpulse verursachten Wirkungen zu messen oder zu ermitteln. Diese Werte werden an die Steuerungseinrichtung 10 übermittelt, die Behandlungsparameter vorschlagen kann und/oder einstellen kann. Darüber hinaus kann mittels der Messeinrichtung der Behandlungsfortschritt überwacht, zumindest ein Parameter in Abhängigkeit des Behandlungsfortschrittes, der aus dem gemessenen oder ermittelten Wert bestimmt wird, angepasst und/oder die Behandlung abgebrochen werden, falls eine Verletzung des Patienten oder eine Schädigung des Gewebes zu befürchten ist. Die Steuerungseinrichtung kann die Änderung der Parameter oder den Abbruch der Behandlung automatisch vornehmen oder einen Operateur mit einem Hinweis auf einer Anzeigeeinrichtung informieren.

Die Sensoreinrichtung 22 kann auch eine Abbildungseinrichtung aufweisen, die mittels optischer Kohärenztomografie oder Ultraschall die Haut abbildet. Dadurch ist es möglich, genauere Aussagen über die zu verwendenden Parameter zu treffen und den Behandlungsfortschritt genauer zu überwachen. Auch diese Daten können von der Steuerungseinrichtung 10, wie zuvor beschrieben, für die Auswahl der Parameter der Laserimpulsquelle 12 verwendet werden.

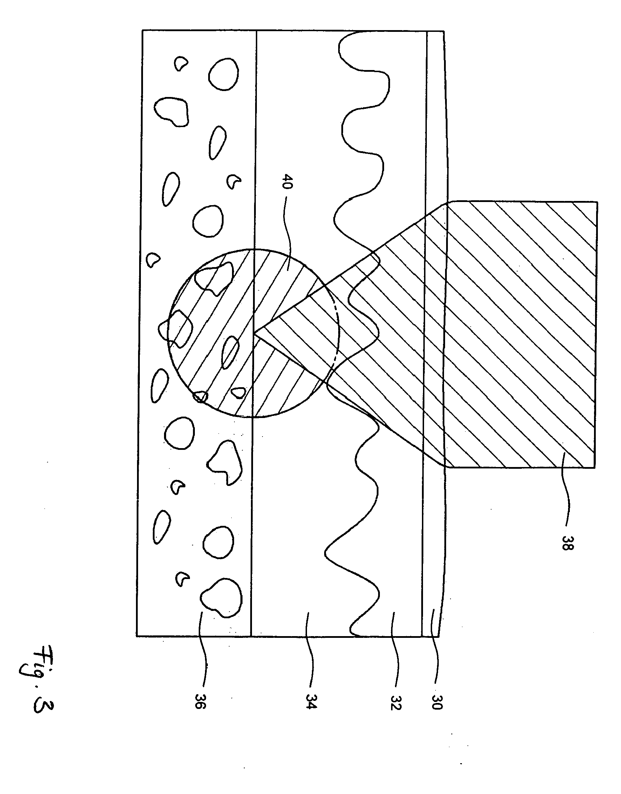

Fig. 3 zeigt einen Schnitt durch die Haut. Die Haut ist detailliert in Roche Lexikon Medizin, 5. Auflage, Hofmann-La Roche AG und Urban & Fischer beschrieben.

Die oberste Hautschicht als Teil der Epidermis ist das Stratum Corneum 30, das aus dem Stratum Disjunctum und dem Stratum Conjunctum besteht (nicht gezeigt). Das Bezugszeichen 32 zeigt die weiteren Schichten der Epidermis. Darunter folgt die Lederhaut (Dermis), die in Stratum Papilare und Stratum Reticulare aufgeteilt ist. Die unterste dargestellte Schicht ist die sogenannte Subkutis, in der sich beispielsweise eine Haarwurzel und subkutanes Fett befindet. Die Epidermis 30, 32 enthält auch natürliche Pigmente. Unnatürliche Pigmente, beispielsweise Tätowierungen, befinden sich üblicherweise in der Lederhaut 34.

Fig. 3 zeigt einen Fall, bei dem ein im wesentlichen paralleler Lichtstrahl 38 in Form eines Laserimpulses auf das Stratum Corneum 30, d. h. den äußeren Schichten der Epidermis, auftrifft. Der Laserimpuls 38 weist eine höhere Intensität als die zuvor beschriebene kritische Leistung $P_k$ auf, oberhalb der die Selbstfokussierung aufgrund des Kerr-Effektes auftritt. Die Brechzahl des Hautgewebes ist folglich eine Funktion der Intensität J der Laser-Impulse. Somit findet eine Selbstfokussierung des Laserimpulses 38 statt.

In Fig. 3 ist dargestellt, dass der Impuls auf einen Punkt am Übergang von der Dermis zum Subkutis fokussiert wird. Der laserinduzierte Durchbruch findet also an der Grenze zwischen Dermis und Subkutis statt. Da der Laserimpuls in der Epidermis 30, 32 eine relativ große Fläche aufweist, ist die Energiedichte des Laserimpulses in der Epidermis 30, 32 relativ niedrig. Folglich findet keine oder keine wesentliche Schädigung der Epidermis 30, 32 statt. Insbesondere das Stratum Corneum wird nicht geschädigt, wodurch die Haut als Ganzes vor Infektionen geschützt wird und die Nebenwirkungen für den Patienten möglichst gering gehalten werden.

Die Behandlungstiefe, d. h. der Ort des laserinduzierten Durchbruchs 40, kann so eingestellt werden, dass die gewünschte Behandlung, beispielsweise Haarwurzelentfernung, Tätowierungsentfernung oder Pigmententfernung, Fettentfernung, Aknebehandlung, Cellulitisbehandlung, Tumorenfernung, Karzinomentfernung, Hautstraffung, Hautverjüngung, Gefäßverödung, Entfernung von pigmentierten Läsionen etc., oder eine andere zuvor beschriebene Behandlung durchgeführt werden kann. In dem in Fig. 3 gezeigten Beispiel kann beispielsweise eine Haarwurzelentfernung oder eine Fettentfernung durchgeführt werden.

Die Laserimpulse können in einer Tiefe von etwa 30 μm bis etwa 10 mm unterhalb der Oberfläche der Haut fokussiert werden, um dort die laserinduzierten Durchbrüche bzw. Photodisruptionen auszulösen. Beispielsweise ist die Epidermis am Augenlied lediglich etwa 30 $\mu$m und an der Fußsohle etwa 4 mm dick.

Die Fleckgröße bzw. Querschnittsfläche der Impulse auf der Oberfläche des Organs kann je nach Pulsdauer und Fokussierungstiefe variieren. Beim der Fokussierung durch den Kerr-Effekt beträgt der Durchmesser bzw. Querschnitt des Flecks bzw. der Querschnittsfläche auf der Oberfläche des Organs zwischen etwa 50$\mu$m und etwa 1mm. Der Fleck bzw. die Querschnittsfläche des Impulses müssen nicht rund sein.

Der Ort des laserinduzierten Durchbruchs 40 kann durch die Intensität des Laserstrahls 38, da der Brechungsindex eine Funktion der Intensität ist, und durch die Größe des Lichtflecks eingestellt werden, da mit zunehmender Größe des Lichtflecks eine längere Strecke in der Haut benötigt wird, um den Laserimpuls 38 soweit zu fokussieren, dass der laserinduzierte optische Durchbruch auftritt.

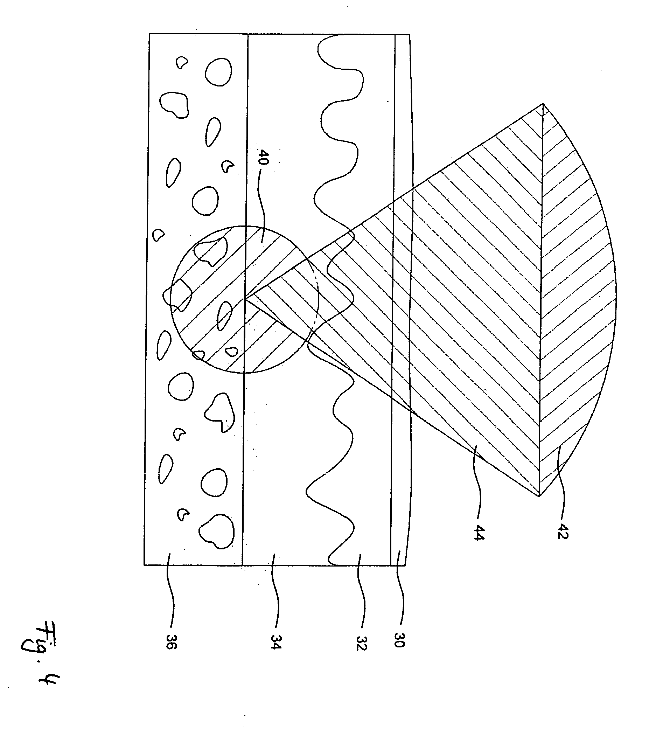

Fig. 4 zeigt eine dritte Ausführungsform der Erfindung. In Fig. 4 ist ein Schnitt durch die Haut dargestellt, der dem Schnitt in Fig. 3 entspricht und folglich wird dessen Beschreibung nicht wiederholt.

Die Ausführungsform gemäß Fig. 4 unterscheidet sich dadurch von der Ausführungsform gemäß Fig. 3, dass eine Fokussierungseinrichtung in Form einer Sammellinse 42, beispielsweise einer konvexen Sammellinse, vorgesehen ist. Die Laserimpulse werden durch die Sammellinse zu einem fokussierten Strahl 44 gebündelt, der in der Epidermis und Dermis weiter gebündelt wird. Dadurch müssen die Impulse nicht eine so hohe Intensität aufweisen, dass der Kerr-Effekt auftritt. Auch mit dieser Ausführungsform können alle zuvor genannten Behandlungen durchgeführt werden.

Die Laserimpulse können in einer Tiefe von etwa 30 $\mu$m bis etwa 10 mm unterhalb der Oberfläche des Organs fokussiert werden, um dort die laserinduzierten Durchbrüche bzw. Photodisruptionen auszulösen. Beispielsweise ist die Epidermis am Augenlied lediglich etwa 30 $\mu$m und an der Fußsohle etwa 4 mm dick.

Bei der Fokussierung durch die Sammellinse beträgt der Durchmesser bzw. Querschnitt des Flecks bzw. der Querschnittsfläche auf der Oberfläche des Organs zwischen etwa 50 $\mu$m und etwa 5 cm. Der Fleck bzw. die Querschnittsfläche des Impulses müssen nicht rund sein.

Die Merkmale der ersten, zweiten und dritten Ausführungsform können miteinander kombiniert werden.

Die vorliegende Erfindung hat den Vorteil, dass das Gewebe in einer definierten Tiefe der Haut, des Zahnfleisches, des Zahns oder eines inneren Organes durch eine Photodisruption verändert werden kann, die durch einen laserinduzierten optischen Durchbruch ausgelöst wird. Die Erfindung hat ferner den Vorteil, dass obere Gewebeschichten der Haut, des Zahnfleisches, des Zahns oder des inneren Organes nicht beschädigt werden, so dass der Heilungsprozess beschleunigt wird und die Nebenwirkungen für den Patienten reduziert werden.

**Patentansprüche**

1. Hautbehandlungsvorrichtung, mit

   - einer Laserimpulsquelle (12) zum Erzeugen von Laserimpulsen; und
   - einem optischen Pfad (14) zum Richten der Laserimpulse von der Laserimpulsquelle auf die Haut;
   **dadurch gekennzeichnet, dass** die Vorrichtung so eingerichtet ist, dass die Laserimpulse so auf die Haut (30 - 36) appliziert werden, dass die Impulse in der Haut (30 - 36) fokussiert werden, so dass sie unter der Hautoberfläche einen laserinduzierten optischen Durchbruch (40) erzeugen, wobei die Laserimpulse an der Oberfläche der Haut (30 - 36) eine größere Querschnittsfläche als am Ort des laserinduzierten optischen Durchbruchs (40) aufweisen.

2. Dentalbehandlungsvorrichtung, mit

   - einer Laserimpulsquelle (12) zum Erzeugen von Laserimpulsen; und
   - einem optischen Pfad (14) zum Richten der Laserimpulse von der Laserimpulsquelle auf das Zahnfleisch oder einen Zahn;
   **dadurch gekennzeichnet, dass** die Vorrichtung so eingerichtet ist, dass die Laserimpulse so auf das Zahnfleisch oder den Zahn appliziert werden, dass die Impulse im Zahnfleisch oder im Zahn fokussiert werden, so dass sie unter der Zahnfleischoberfläche oder unter der Zahnoberfläche einen laserinduzierten optischen Durchbruch (40) erzeugen, wobei die Laserimpulse an der Oberfläche des Zahnfleisches oder des Zahns eine größere Querschnittsfläche als am Ort des laserinduzierten optischen Durchbruchs (40) aufweisen.

3. Chirurgische Vorrichtung zur Behandlung eines inneren Organs, mit

   - einer Laserimpulsquelle (12) zum Erzeugen von Laserimpulsen; und
   - einem optischen Pfad (14) zum Richten der Laserimpulse von der Laserimpulsquelle auf die Oberfläche eines inneren Organs;

**dadurch gekennzeichnet, dass** die Vorrichtung so eingerichtet ist, dass die Laserimpulse so auf die Oberfläche eines inneren Organs appliziert werden, dass die Impulse in dem inneren Organ fokussiert werden, so dass sie unter der Oberfläche des inneren Organs einen laserinduzierten optischen Durchbruch erzeugen, wobei die Laser-impulse an der Oberfläche des inneren Organs eine größere Querschnittsfläche als am Ort des laserinduzierten optischen Durchbruchs aufweisen.

4. Vorrichtung nach einem der Ansprüche 1 bis 3, **gekennzeichnet durch** eine Fokussierungseinrichtung (42) im optischen Pfad, die so eingerichtet ist, dass die Laserimpulse (44) auf der Strecke von der Oberfläche der Haut, des Zahnfleisches, des Zahns oder des inneren Organs zum Ort des laserinduzierten Durchbruchs (40) fokussiert werden.

5. Vorrichtung nach einem der Ansprüche 1 bis 3, **dadurch gekennzeichnet, dass** die Vorrichtung so eingerichtet ist, dass die Laserimpulse (38) beim Eintritt in die Haut, in das Zahnfleisch, in den Zahn oder in das Organ eine Intensität aufweisen, die höher als ein Selbstfokussierungsschwellenwert des Hautgewebes, des Zahnfleischgewebes, des Zahns oder des Gewebes des inneren Organs ist, so dass in der Haut, im Zahnfleisch, im Zahn oder im inneren Organ eine Selbstfokussierung auftritt, um den laserinduzierten optischen Durchbruch (40) unter der Hautoberfläche, im Zahnfleisch, im Zahn oder im inneren Organ zu ermöglichen.

6. Vorrichtung nach einem der Ansprüche 1 bis 5, **dadurch gekennzeichnet, dass** die von der Laserimpulsquelle (12) abgegebenen Impulse eine Dauer von etwa 1 fs bis etwa 1000 ps, vorzugsweise von etwa 10 fs bis etwa 100 ps, vorzugsweise von etwa 50 fs bis etwa 10 ps, höchst vorzugsweise von etwa 50 fs bis etwa 900 fs aufweisen.

7. Vorrichtung nach einem der Ansprüche 1 bis 6, **dadurch gekennzeichnet, dass** die von der Laserimpulsquelle (12) abgegebenen Impulse eine Wellenlänge von etwa 400 nm bis etwa 10000 nm, vorzugsweise von etwa 700 nm bis etwa 2000 nm, höchst vorzugsweise von etwa 800 nm bis etwa 1500 nm, höchst vorzugsweise von etwa 950 nm bis etwa 1400 nm aufweisen.

8. Vorrichtung nach einem der Ansprüche 1 bis 7, **dadurch gekennzeichnet, dass** der optische Pfad (14) eine optische Faser, insbesondere eine hohle optische Faser oder eine photonische Kristallfaser, aufweist.

9. Vorrichtung nach einem der Ansprüche 1 bis 8, **dadurch gekennzeichnet, dass** der optische Pfad (14) eine Scaneinrichtung aufweist, um die Laserimpulse auf einer definierten Fläche zu applizieren.

10. Vorrichtung nach einem der Ansprüche 1 bis 9, **gekennzeichnet durch** eine Messeinrichtung, die dazu ausgebildet ist, Eigenschaften der Haut (30 - 36), insbesondere den Hauttyp, die Hauttemperatur und/oder die in der Haut, im Zahnfleisch, im Zahn oder im inneren Organ **durch** die Laserimpulse verursachten Wirkungen zu messen oder zu ermitteln.

11. Vorrichtung nach einem der Ansprüche 1 bis 10, **gekennzeichnet durch** eine Abbildungseinrichtung, die mittels optischer Kohärenztomographie oder Ultraschall die Haut abbildet.

12. Vorrichtung nach einem der Ansprüche 1 bis 11, **gekennzeichnet durch** eine Kühleinrichtung zum Kühlen der Haut vor und/oder während und/oder nach der Behandlung.

13. Vorrichtung nach einem der Ansprüche 1 bis 12, **gekennzeichnet durch** eine Unterdruckerzeugungseinrichtung zum Erzeugen eines Unterdrucks auf der Oberfläche der Haut (30 - 36), des Zahnfleisches, des Zahns oder des inneren Organs, um deren Position festzulegen und/oder sie zu strecken.

14. Vorrichtung nach einem der Ansprüche 1 bis 13, **gekennzeichnet durch** eine Positionierungseinrichtung, die dazu ausgebildet ist, die Laserimpulse (38, 44) so in der Haut (30 - 36), im Zahnfleisch, im Zahn oder im inneren Organ zu positionieren, dass die Orte der laserinduzierten Durchbrüche (40) einander benachbart sind oder sich überlappen.

15. Vorrichtung nach einem der Ansprüche 1 bis 13, **gekennzeichnet durch** eine Positionierungseinrichtung, die dazu ausgebildet ist, die Laserimpulse (38, 44) so in der Haut (30 - 36), im Zahnfleisch, im Zahn oder im inneren Organ zu positionieren, dass zwischen den Orten der laserinduzierten Durchbrüche (40) nicht mit Laserimpulsen behan-deltes Gewebe vorhanden ist.

Fig. 1

Fig. 2

Fig. 3

Fig. 4

36

34

40

32

30

44

42

EP 2 366 355 A1

12

Europäisches Patentamt
European Patent Office
Office européen des brevets

## EUROPÄISCHER RECHERCHENBERICHT

Nummer der Anmeldung

EP 10 00 2770

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2008/089344 A2 (NEEV JOSEPH [US]) 24. Juli 2008 (2008-07-24) * Absätze [0031], [0032], [0033], [0160], [0194], [0213]; Abbildungen 1,2a * | 1-4,6-15 | INV. A61B18/20 A61C1/00 |
| X | US 2008/172047 A1 (ALTSHULER GREGORY B [US] ET AL) 17. Juli 2008 (2008-07-17) * Absätze [0102], [0106], [0180]; Abbildungen 1,2,11a * | 1-4,6-15 | |
| X | US 2009/149843 A1 (SMITS ALEXANDER [US] ET AL) 11. Juni 2009 (2009-06-11) * Absätze [0029], [0034]; Abbildungen 1,2 * | 1-4,6-8 | |
| X | WO 2005/037234 A2 (HOMER GREGG S [US]) 28. April 2005 (2005-04-28) * Absatz [0032]; Anspruch 44; Abbildung 8 * | 1-5,8,9 | |
| X | US 6 106 514 A (O'DONNELL JR FRANCIS E [US]) 22. August 2000 (2000-08-22) * Spalte 4, Zeile 30 - Zeile 40; Abbildung 4 * | 1-4,8, 10,12 | RECHERCHIERTE SACHGEBIETE (IPC) A61B A61C |
| X | WO 2007/038567 A1 (CANDELA CORP [US]; JONES CHRISTOPHER J [US]; HSIA JAMES C [US]; PAITHA) 5. April 2007 (2007-04-05) * Absätze [0018], [0042], [0049]; Abbildungen 3,4 * | 1-4,7-15 | |
| X | US 5 112 328 A (TABOADA JOHN [US] ET AL) 12. Mai 1992 (1992-05-12) * Spalte 8, Zeile 10 - Zeile 18; Abbildungen 2,3 * | 2 | |

Der vorliegende Recherchenbericht wurde für alle Patentansprüche erstellt

| Recherchenort | Abschlußdatum der Recherche | Prüfer |
|---|---|---|
| Den Haag | 25. August 2010 | Mayer-Martenson, E |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

......................................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 10 00 2770

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-08-2010

| Im Recherchenbericht angeführtes Patentdokument | | Datum der Veröffentlichung | Mitglied(er) der Patentfamilie | | Datum der Veröffentlichung |
|---|---|---|---|---|---|
| WO 2008089344 | A2 | 24-07-2008 | EP | 2111251 A2 | 28-10-2009 |
| US 2008172047 | A1 | 17-07-2008 | US | 2008306471 A1 | 11-12-2008 |
| | | | US | 2008183162 A1 | 31-07-2008 |
| | | | US | 2008214988 A1 | 04-09-2008 |
| | | | WO | 2008083305 A2 | 10-07-2008 |
| US 2009149843 | A1 | 11-06-2009 | KEINE | | |
| WO 2005037234 | A2 | 28-04-2005 | CA | 2535475 A1 | 28-04-2005 |
| | | | EP | 1680038 A2 | 19-07-2006 |
| | | | MX | PA06003466 A | 05-06-2006 |
| | | | US | 2005080466 A1 | 14-04-2005 |
| US 6106514 | A | 22-08-2000 | US | 6197020 B1 | 06-03-2001 |
| WO 2007038567 | A1 | 05-04-2007 | EP | 1928549 A1 | 11-06-2008 |
| US 5112328 | A | 12-05-1992 | KEINE | | |

Für nähere Einzelheiten zu diesem Anhang : siehe Amtsblatt des Europäischen Patentamts, Nr.12/82

EPO FORM P0461

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7131968 B2

**In der Beschreibung aufgeführte Nicht-Patentliteratur**

- **BERGMANN SCHÄFER.** Lehrbuch der Experimentalphysik. vol. 3, 940 ff

- **BERGMANN SCHÄFER.** Lehrbuch der Experimentalphysik. vol. 3